(19) Europäisches Patentamt
European Patent Office
Office européen des brevets

(11) EP 0 707 072 A1

(12) **DEMANDE DE BREVET EUROPEEN**

(43) Date de publication:
**17.04.1996 Bulletin 1996/16**

(51) Int Cl.6: **C12P 7/26**, C12P 7/22, C11B 9/00

(21) Numéro de dépôt: **95402114.3**

(22) Date de dépôt: **19.09.1995**

(84) Etats contractants désignés:
**AT BE CH DE DK ES FR GB GR IE IT LI LU MC NL PT SE**

(30) Priorité: **19.09.1994 FR 9411142**

(71) Demandeur: **BFA LABORATOIRES**
**F-06117 Le Cannet Cédex (FR)**

(72) Inventeurs:
- **Dumont, Benoît**
  **F-63430 Pont-du-Chateau (FR)**
- **Hugueny, Patricia**
  **F-68200 Mulhouse (FR)**
- **Belin, Jean-Marc**
  **F-21121 Fontaine-Lès-Dijon (FR)**

(74) Mandataire: **Almond-Martin, Carol et al**
**Ernest Gutmann - Yves Plasseraud S.A.**
**3, rue Chauveau-Lagarde**
**F-75008 Paris (FR)**

(54) **Production par bioconversion de cétone framboise**

(57) L'invention concerne un procédé de fabrication, par voie enzymatique, de 4-(4'-hydroxyphényl)butan-2-one ("cétone framboise") caractérisé par la mise en contact du 4-(4'-hydroxyphényl)butan-2-ol avec une source d'enzyme ayant une activité d'alcool déshydrogénase.

Le produit de l'invention est utilisé comme substance naturelle d'arôme dans des produits alimentaires, cosmétiques, pharmaceutiques ou dans des parfums.

EP 0 707 072 A1

## Description

L'invention concerne un procédé pour la fabrication, par bioconversion, (voie microbienne ou voie enzymatique) de la 4-(4'-hydroxyphényl)butan-2-one ou "cétone framboise". L'invention concerne également le produit obtenu par le bioprocédé et son utilisation comme substance d'arôme, par exemple "fruits rouges", dans des produits alimentaires, pharmaceutiques et cosmétiques.

La 4-(4'-hydroxyphényl)butan-2-one est un composé important de l'arôme de framboise auquel il apporte une note de fond. Il est présent dans un nombre restreint de fruits et végétaux, notamment dans les fruits de framboise (Rubus idaeus), d'airelle (Vaccinium oxycoccus), d'argousier (Hippophae rhamnoides), dans les racines de rhubarbe (Rheum palmatum), dans les aiguilles de pin (Pinus contorta), dans les parties aériennes de scutellaire (Scutellaria rivularis), et d'Artemisia hispanica, dans la pêche (fruit de Prunus persica), et dans les fleurs d'une orchidée, Dendrobium superbum. Ce composé est également connu sous les noms de 4-(para-hydroxyphényl)butan-2-one, rasketone, p-hydroxybenzyl acetone, para-hydroxyphényléthyl méthyl cétone, 1-(4'-hydroxyphényl)butan-3-one, (Clark, G.S., 1992, Perfumer and Flavorist, 17, p 21-26). Il existe aussi en synthèse et porte alors les noms de raspberry ketone, Frambinone, Oxananone ou oxyphenylon.

A ce jour, il n'existe pas de substitut pour la cétone framboise. En effet, celle-ci est le seul composé chimique capable de conférer véritablement l'arôme caractéristique de framboise, Actuellement, la totalité de la cétone framboise utilisée comme arôme est fabriquée par voie chimique, par exemple par la condensation de p-hydroxy-benzaldéhyde avec de l'acétone. Le produit ainsi obtenu est un arôme "identique-nature", c'est-à-dire qu'il s'agit d'une molécule obtenue par synthèse chimique ou isolée par des procédés chimiques et identique chimiquement a une substance présente naturellement dans une matière d'origine végétale ou animale. Il n'existe aucune source naturelle de cétone framboise exploitable du point de vue commercial, l'extraction et la purification du produit naturel n'étant guère envisageables en raison des faibles concentrations présentes dans les framboises (0.1- 2.0 ppm).

La fabrication de cétone framboise par bioconversion n'a jamais été décrite. De plus, très peu d'informations sont disponibles en ce qui concerne la voie métabolique naturelle d'obtention de la cétone framboise. Certains auteurs ont suggéré que chez la framboise, la cétone est synthétisée à partir de l'acide coumarique (Deifel A., 1989, Z. Lebensm. Unters. Forsch., 188 : 330-332) mais cette hypothèse reste à démontrer. En outre, la présence chez la framboise de certains glucosides de cétone framboise (notamment le 4-(4'-hydroxyphényl)butan-2-one 4'-O-β D-glucopyranoside et le 4-(3',4'-dihydroxyphényl)butan-2-one 3'-O-β-glucopyranoside) (Pabst A. et al., 1990, Phytochemistry, 29 (12) : 3853-3858) suggère que ces glucosides pourraient intervenir dans le métabolisme de la cétone framboise.

Le problème technique que se propose de résoudre la présente invention est l'identification d'un "chaînon métabolique" pour la fabrication de la cétone framboise. Ce dernier doit permettre l'utilisation de matières premières peu coûteuses et faciles à obtenir, et doit aboutir à des rendements de cétone framboise exploitables a l'échelle industrielle.

Les présente inventeurs ont résolu ce problème technique tout d'abord en identifiant un substrat, le 4-(4'-hydroxyphényl)butan-2-ol (également appelé "bétuligénol" ou "rhododendrol") qui peut être transformé, par une source d'enzyme ayant une activité alcool déshydrogénase, en cétone framboise. Le succès et l'efficacité de cette transformation sont surprenants : le 4-(4'-hydroxyphényl)butan-2-ol n'a pas été retrouvé, à ce jour, dans la framboise ni au sein des autres espèces contenant la cétone framboise. Une conversion enzymatique a donc été identifiée dans le cadre de la présente demande. En outre, le 4-(4'-hydroxyphényl)butan-2-ol n'a jamais été utilisé comme substrat avec une enzyme ayant une activité alcool déshydrogénase. De manière surprenante, les inventeurs ont démontré qu'un grand nombre d'alcools déshydrogénases (et en particulier, un grand nombre de microorganismes), sont capables d'effectuer la déshydrogénation du 4-(4'-hydroxyphényl)butan-2-ol en cétone framboise avec des rendements exploitables a l'échelle industrielle. De préférence, il s'agit d'une alcool secondaire déshydrogénase.

L'invention concerne donc un procédé de fabrication, par bioconversion, de 4-(4'-hydroxyphenyl)butan-2-one (cétone framboise) caractérisé par la mise en contact du 4-(4'-hydroxyphényl)butan-2-ol avec une source d'enzyme ayant une activité alcool déshydrogénase.

Dans le contexte de l'invention, la désignation "cétone framboise" signifie la 4-(4'-hydroxyphényl)butan-2-one (voir schéma figure 1). Ce composé de poids molaire 164 est une poudre cristalline blanche, avec un point de fusion de 82 à 83°C, soluble, entre autres, dans l'acétone, l'acétonitrile, l'ethanol, l'éther diéthylique, le méthanol, les esters, le DEP. Elle est faiblement soluble voire insoluble dans l'eau, l'éther de pétrole, l'hexane, les hydrocarbures et les solvants chlorés.

Selon l'invention, le substrat de la réaction enzymatique est le 4-(4'-hydroxyphényl)butan-2-ol (figure 1, deuxième étape). Cet alcool secondaire possède un atome de carbone asymétrique (C2) ; il existe deux énantiomères, le (+)-4-(4'-hydroxyphényl)butan-2-ol et le (-)-4-(4'-hydroxyphényl)butan-2-ol (Das B. et al., 1993, Phytochemistry, 33 (6) : 1529-1530 ; Virinder S. et al., 1991, J. Chem. Soc. Perkin. Trans. 1, 2487-2490) (voir figure 2). Ces composés sont également connus sous le nom (+)rhododendrol et (-)rhododendrol, respectivement. Le "bétuligénol" tend à être identifié au (-)rhododendrol par différents auteurs. Le (+)rhododendrol est généralement S, le (-)rhododendrol serait R.

Dans le contexte de l'invention, et conformément à la convention, le terme "bétuligénol" est utilisé pour désigner le

(-)-4-(4'-hydroxyphényl)butan-2-ol, et le terme (+)rhododendrol est utilisé pour désigner le (+)-4-(4'-hydroxyphényl)butan-2-ol.

Selon l'invention, chacun des énantiomères peut être utilisé comme substrat pour la réaction enzymatique, ainsi que des mélanges contenant les deux formes. L'utilisation du bétuligénol est cependant préférée.

Selon l'invention, le 4-(4'-hydroxyphényl)butan-2-ol est de préférence obtenu par fabrication enzymatique (voir exemples ci-dessous). Néanmoins, bien que la voie enzymatique soit préférée, il est aussi possible, dans le cadre de l'invention, d'obtenir le substrat par d'autres moyens, par exemple par voie chimique (Zemplen G. et al., 1944, Ber. 77 B, 784 ; Das B. et al., supra) ou par extraction à partir de sources végétales, par exemple à partir de plantes apparterant à l'une des familles suivantes: Betulaceae, Ericacea, Saxifragaceae ou à partir de Taxus baccata (famille des Taxaceae), Acer nikoense (famille des Aceraceae), Cistus palinhae (famille des Cistaceae), Abies webbiana (famille des Abietaceae), ou Cotyledon wallichii (famille des Crassulaceae). L'espèce végétale utilisée comme source de 4-(4'-hydroxyphényl)-butan-2-ol détermine l'activité optique du substrat, le bétuligénol se trouvant chez de nombreuses espèces, et le (+)rhododendrol se trouvant chez Rhododendron maximum et Acer nikoense MAXIM.

Le 4-(4'-hydroxyphényl)butan-2-ol est mis en contact avec une source d'enzyme ayant une "activité alcool déshydrogénase" dans des conditions opératoires permettart la transformation du substrat en cétone framboise. L'expression "activité alcool déshydrogénase" signifie dans le contexte de l'invention que la préparation enzymatique est capable d'effectuer la déshydrogénation du bétuligénol ou du (+)rhododendrol pour donner la cétone framboise. Normalement, le rendement molaire de bioconversion est d'au moins 10%, et de préférence compris entre 30 et 100%. Ce rendement est obtenu après au moins 20 heures de réaction, par exemple 36 à 200 heures. L'obtention de cétone framboise dans le milieu peut être contrôlée par chromatographie liquide haute performance (CLHP) voire par chromatographie couche mince (CCM) (voir Gallois, A., 1982, Sciences des Aliments, 2, 99-106).

Les conditions opératoires sous lesquelles la réaction enzymatique se déroule varient en fonction de la source de l'enzyme présentant l'activité alcool déshydrogénase, mais en général, la réaction est effectuée entre 20 et 36°C, par exemple entre 20 et 30°C, pour une durée d'au moins 20 heures, de préférence au moins 24 heures, sous agitation dans un milieu aéré.

Comme source d'enzyme ayant une activité alcool déshydrogénase, on peut utiliser des sources microbiennes ou encore des sources végétales ou animales.

Par "source microbienne", il faut comprendre soit une culture de micro-organismes, notamment bactéries, levures ou champignons, soit un extrait ou un dérivé d'un tel micro-organisme, par exemple un lyophilisat ou homogénat, soit encore une enzyme purifiée à partir d'un tel micro-organisme.

Le terme "source végétale" comprend soit une culture de cellules végétales présentant une activité alcool déshydrogénase par exemple un cal, soit un tissu végétal par exemple pétiole, feuille, etc.., soit un extrait ou dérivé d'un tissu végétal, soit une enzyme purifiée à partir d'une plante.

L'activité alcool déshydrogénase de ces différentes sources peut être vérifiée en les mettant en contact avec le substrat, suivi par la détection de la production de cétone framboise comme indiqué ci-dessus.

De préférence, la source de l'enzyme ayant une activité alcool déshydrogénase est une source microbienne. Les bactéries sont particulièrement préférées, par exemple celles appartenant aux genres suivants :Acetobacter, Gluconobacter, Mycoderma, Pseudomonas, Bacterium, Corynebacterium, Rhodopseudomonas, Astaria, Thermoanaerobium, Clostridium, Methanobacterium, Methylobacterium, Methylococcus, Methylosinus, Lactobacillus, Methanocorpusculum, Methanomicrobium, Methanogenium, Thermoanaerobacter, Acinetobacter, Comamonas, Aerobacter, Torulopsis, Escherichia par exemple Escherichia coli, soit un extrait ou une fraction de bactéries appartenant a l'un ou plusieurs de ces genres. Comme exemples, on peut citer Acetobacter aceti, Acetobacter xylinum, Acetobacter pasteurianus, Pseudomonas putida (par exemple ATCC 12633, anciennement Pseudonomas fluorescens), Pseudomonas sp. (ATCC 21439), Bacterium (pare exemple N.C.I.B. 8250), Corynebacterium equi (par exemple I.F.O. 3730), Rhodopseudomonas acidophila, Astasia longa, Thermoanaerobium brockii, Clostridium thermohydrosulfuricum, Methanobacterium palustre, Methylobacterium organophilum, Methylococcus capsulatus, Methylosinus trichosporium, Lactobacillus arabinosus, Rhodococcus equi.

La source microbienne de l'enzyme ayant une activité alcool déshydrogénase peut également être une levure, particulièrement celles appartenant aux genres Lipomyces, Hansenula, Pichia, Candida, Oospora, Saccharomyces, soit un extrait de levure appartenant à l'un ou plusieurs de ces genres. Comme exemples de levures particulièrement avantageuses on peut citer Lipomyces starkeyi (par exemple ATCC 12659), Hansenula polymorpha (par exemple ATCC 26012), Hansenula wickerhamii, Pichia sp. (NRRL-Y-11328), Candida boidinii, Candida krusei, Candida utilis, Oospora suaveolens, Saccharomyces cerevisiae.

Parmi d'autres sources d'enzymes ayant une activité alcool déshydrogénase, on peut citer les champignons, en particulier ceux appartenant au genre Neurospora, par exemple N. crassa.

Parmi ces différentes sources microbiennes, Acetobacter, Candida, Rhodococcus et Pseudomonas sont particulièrement préférés.

Comme source végétale d'enzyme ayant une activité alcool déshydrogénase, on peut citer l'érable Acer nikoense.

Comme source animale, on peut citer un extrait de foie.

L'enzyme ayant une activité alcool déshydrogénase est typiquement une enzyme appartenant à la sous-classe des oxydo-réductases agissant sur les groupements CHOH (classification E.C. 1.1.-.-, par exemple une alcool déshydrogénase a NAD = E.C. 1.1.1.1., ou encore, une alcool déshydrogénase a NADP = E.C. 1.1.1.2). Il peut s'agir d'une alcool déshydrogénase proprement dite , ou de toute enzyme présentant, outre son activité enzymatique principale, une activité alcool déshydrogénase comme activité secondaire. Normalement, il s'agit d'une alcool déshydrogénase capable d'effectuer la déshydrogénation d'alcools secondaires avec un cycle aromatique, mais il est également possible d'utiliser des alcools déshydrogenases connues pour leur action sur des alcools secondaires non aromatiques. Une alcool primaire déshydrogénase peut aussi convenir, à condition qu'elle soit capable de catalyser la réaction.

Lorsque la source de l'enzyme est un micro-organisme ou une source végétale ou un extrait microbien ou végétal, l'activité enzymatique peut être dûe à une seule enzyme, ou peut être dûe à plusieurs enzymes, chacune d'elles ayant une activité alcool déshydrogénase ou ayant une activité enzymatique qui, lorsqu'elles sont mises en commun, confèrent une activité alcool déshydrogénase.

La source d'alcool déshydrogénase peut également être une enzyme purifiée à partir d'une source microbienne ou végétale. Par "purifiée", il faut comprendre que la préparation enzymatique est dépourvue de toute autre activité enzymatique capable, dans les conditions utilisées, de catalyser la production de substances autres que la cétone framboise, et que la préparation est telle qu'elle évite la formation de produits secondaires.

La source d'alcool déshydrogénase peut également être un mélange d'au moins deux sources telles que définies ci-dessus.

Il se peut que la préparation enzymatique ayant une activité alcool déshydrogénase soit spécifique d'un énantiomère du 4-(4'-hydroxyphényl)butan-2-ol. Dans ce cas, il est bien entendu que l'enzyme utilisée doit être capable d'effectuer la déshydrogénation de l'énantiomère choisi comme substrat.

Selon une variante particulièrement préférée de l'invention, le substrat de la réaction de déshydrogénation, c'est-à-dire le 4-(4'-hydroxyphényl)butan-2-ol, est obtenu par voie enzymatique à partir du 3-(4'-hydroxyphényl)-1-méthyl-propyl-β-D-glucopyranoside également connu sous les noms "bétuloside" et "rhododendrine". Selon cette variante de l'invention, le bétuloside/rhododendrine est mis en contact avec une préparation enzymatique ayant une activité β-glucosidase, Cette préparation catalyse l'hydrolyse du substrat.

Le procédé de fabrication de cétone framboise, selon l'invention, comporte alors deux étapes : une étape d'hydrolyse du bétuloside par l'enzyme ayant une activité β-glucosidase et une étape de déshydrogénation telle que décrite ci-dessus (voir figure 1).

Il est à noter qu'il existe deux formes du 3-(4'-hydroxyphényl)-1-méthyl-propyl-β-D-glucopyranoside : on distingue actuellement la rhododendrine (également connue sous le nom de "bétuloside") et l'épi-rhododendrine (voir figure 2). Dans le contexte de la présente invention, les termes "bétuloside" et "rhododendrine" sont utilisés pour signifier la molécule ayant la conformation indiquée dans la figure 2. Le terme 3-(4'-hydroxyphényl)-1-méthyl-propyl-β-D- glucopyranoside (ou simplement "glucoside") est utilisé pour englober la rhododendrine et l'épirhododendrine.

La plupart des espèces végétales contiennent le bétuloside (souvent accompagné du bétuligénol ou (-)rhododendrol), mais il a été constaté que Rhododendron maximum et Acer nikoense contiennent l'épi-rhododendrine et son aglucone le (+)rhododendrol. Les deux éniantiomères peuvent être utilisés selon l'invention, mais le bétuloside est préféré.

Selon cette variante de l'invention, le substrat, c'est-à-dire le bétuloside (ou rhododendrine) est de préférence obtenu par extraction d'une matière végétale.

Comme exemple de sources de bétuloside, on peut citer :

i) toutes les espèces naturelles ou sélectionnées de bouleaux (Betulaceae), par exemple Betula alba, B. alleghaniensis, B. uber, B. albo-sinensis var. septentrionalis, B. costata, B. ermanii var. subcordata, B. jacquemontiana, B. nana, B. pumila L. B. davurica, B. papyrifera, B. pendula, B. platyphylla var. japonica, B. populifolia, B. pubescens, B. luminifera, B. maximoxicziana, B. verrucosa. D'après certains auteurs, B. alba est identique à B. pendula et à B. verrucosa.

ii) toutes les espèces naturelles ou sélectionnées de rhododendron (Ericaceae), par exemple ceux de la série ponticum : R. brachycarpum, R. caucasicum, R. makinoi, R. smirnowii, R. catawbiense, R. ponticum, R. catawbiense, R. ponticum, R. Cunnigham's White (hybride : caucasicum x ponticum "Album"), R. chrysanthum, R. maximum ; ceux de la série thomsonii : R. williamsianum ; ceux de la série ferrugineum : R. hirsutum, R. ferrugineum ; ceux de la série virgatum : R. racemosum, R. fauriae var. rufescens, R. degronianum, R. aureum, R. aeruginosum, R. dauricum, R. fastigiatum, R. forrestii repens, R. hyperythrum, R. kaempferi, R. x loderi, R. mucronatum, R. pachytrichum, R. roxicanum, R. soulei, R. scintillans, R. thomsonii, R. dabanshanense, R. agglutinaum.

iii) toutes les espèces et variétés de Bergenia (Saxifragaceae), par exemple B. cortifolia, B. crassifolia.

iv) Alnus glutinosa.

v) Taxus baccata.

vi) Acer nikoense.

vii) Cistus palinhae.

viii) Abies webbiana.

ix) Cotyledon wallichii.

Le bouleau blanc (Betula alba) est particulièrement préféré, l'écorce fraîche prélevée pendant la période hivernale étant la plus riche en glucoside (compris entre 1 et 30% , par exemple entre 1 et 14% ).

Une "activité β-glucosidase" signifie dans le contexte de l'invention, la capacité de la préparation enzymatique d'hydrolyser le bétuloside (ou la rhododendrine) en 4-(4'-hydroxyphényl)butan-2-ol et glucose. Les conditions optimales de la β-glucosidase sont : température de 37°C, pH compris entre 5 et 7. De manière générale, donc, cette étape du procédé de l'invention s'effectue entre 20 et 40°C, par exemple entre 30 et 37°C, à pH 4 à 8, par exemple pH5 à 7 et pour une durée d'au moins 2 heures, plus généralement d'environ 6 heures. La réaction d'hydrolyse peut être suivie par un test enzymatique pour la détermination du glucose (chimie alimentaire - Boehringer Mannheim). Ce test mesure, par l'augmentation de l'absorbance à 340 nm, la quantité de NADPH formé au cours de la réaction, qui est proportionnelle à la quantité de glucose. Il est cependant préférable de suivre la réaction d'hydrolyse par quantification du bétuligénol libéré, par CLHP. La Résonance Magnétique Nucléaire (RMN) a permis de confirmer qu'il s'agissait bien du 4-(4'-hydroxyphényl)butan-2-ol.

La préparation enzymatique ayant une activité de β-glucosidase peut être un micro-organisme ou un tissu végétal, un extrait ou un homogénat de micro-organisme ou de tissu végétal, ou une enzyme purifiée à partir d'un micro-organisme ou d'un tissu végétal. De préférence, il s'agit d'une préparation enzymatique provenant d'une source végétale, par exemple des amandes. Ce type d'enzyme est disponible dans le commerce sous le nom d'Emulsine (β-D-glucoside glucohydrolase EC 3.2.1.21). On peut également utiliser l'Hespéridinase (β-glucosidase d 'Aspergillus niger) et toute préparation enzymatique possédant une activité beta-glucosidase secondaire, comme, par exemple la pectinase d'Aspergillus niger. (Rohapect)

L'étape d'hydrolyse et l'étape de déshydrogénation peuvent s'effectuer de manière simultanée ou de manière successive. Il est préférable de les effectuer de manière successive.

Selon un mode de réalisation particulièrement préféré, le procédé de l'invention comprend les étapes suivantes :

i) extraction du 3-(4'-hydroxyphényl)-1-méthylpropyl-β-D-glucopyranoside à partir d'une source végétale ;

ii) purification de l'extrait brut du glucoside ainsi obtenu :

iii) mise en contact de l'extrait purifié du 3-(4'-hydroxyphényl)-1-méthyl-propyl-β-D-glucopyranoside, de préférence en milieu aqueux, avec une préparation enzymatique ayant une activité β-glucosidase ;

iv) extraction du 4-(4'hydroxyphényl)butan-2-ol ainsi obtenu, de préférence dans une phase organique ;

v) mise en contact du 4-(4'-hydroxyphényl)butan-2-ol avec une source d'alcool déshydrogénase ;

vi) extraction de la cétone framboise ainsi obtenue.

La phase organique est choisie pour respecter la réglementation en vigueur relative aux produits alimentaires en particulier s'agissant de l'appellation "naturel".

L'extraction du 3-(4'-hydroxyphényl)-1-méthylpropyl-β-D-glucopyranoside à partir de la source végétale (broyée pour donner lieu à des morceaux de taille inférieure à 1.5 cm) s'effectue normalement à l'aide d'eau a 100°C, suivie éventuellement d'une étape de purification partielle avec l'oxyde de magnésium et de l'alcool chaud (par exemple alcool éthylique à 95°) (voir Sosa A., 1933, C. R. Acad. Sc. Paris, 196 : 1827-1830 ; Sosa A. et Sosa-Bourdouil C., 1936, Bulletin de la Société de Chimie Biologique, 18 : 918-925). Normalement, cette étape d'extraction a une durée comprise entre 2 et 36 heures (par exemple 12 à 36 heures) et conduit à l'obtention d'environ 1 à 10 g de glucoside (pur) par kg de matière végétale fraîche, selon la source. La pureté du glucoside est estimée entre 10 et 20% (p/p), par exemple entre 10 et 15% (p/p). Elle peut atteindre 30 à 40% (p/p). Souvent, l'extrait contient non seulement le glucoside, mais également l'aglucone correspondant, c'est-à-dire le bétuligénol ou le (+)rhododendrol. Parmi les impuretés, on peut constater la présence de tanins, de sucres, de graisses, de cires, de résines, de phytostérols, d'acides gras et de composés condensés divers.

L'extrait brut est ensuite soumis à une étape de purification, par exemple sur membrane, ou par chromatographie en phase inverse. La purification sur membrane est particulièrement préférée et conduit à une pureté d'environ 30% de glucoside (voir exemples ci-dessous : technique de nanofiltration).

L'extrait purifié est alors mis en contact, de préférence en milieu aqueux, avec la préparation enzymatique ayant une activité β-glucosidase. Cette étape est normalement effectuée a une température comprise entre 20 et 40°C à pH 4 à 8, et pour une durée d'au moins 2 heures, dans une cuve agitée, thermostatée.

Le ratio enzyme / substrat est normalement de 10 à 2000 mg/g en fonction de la nature et de l'activité de l'enzyme, avantageusement de 10 à 1000 mg/g et, en particulier de 10 à 500 mg/g et de façon intéressante de 10 à 30 mg/g. Par exemple dans le cas de l'Emulsine, le ratio enzyme / substrat est de 19,5 mg/g environ, lorsque l'activité de l'enzyme est d'environ 6,9 U/mg. Lorsque l'enzyme est le Rohapect, le ratio enzyme / substrat pourra être de 600 mg/g environ. Cette étape permet l'obtention de bétuligénol/(+)rhododendrol avec un rendement molaire de 60 à 100%, par exemple d'environ 90%, l'alcool secondaire étant alors de préférence extrait de la phase aqueuse par une phase organique, par exemple l'éther diéthylique, et séché pour donner lieu à un résidu sec (pureté en bétuligénol ou (+)rhododendrol environ 50 à 60%).

Le bétuligénol et/ou (+)rhododendrol ainsi obtenu est ensuite mis en contact avec une enzyme ayant une activité alcool déshydrogénase dans des conditions telles que décrites ci-dessus. Si la source de l'enzyme est un micro-organisme, le milieu réactionnel doit être adapté au micro-organisme utilisé.

La cétone framboise obtenue est finalement extraite par la réalisation d'une extraction liquide/liquide, par exemple dans une phase organique, tel que l'éther diéthylique ou des esters tels que l'acétate d'éthyle. Le solvant est normalement acceptable sur le plan du "naturel". On peut également effectuer l'extraction par chromatographie à phase inverse ou par cyclodextrines. Le taux de conversion obtenu est au moins 60% de l'intermédiaire (alcool secondaire), de préférence 80 à 100%, et la pureté du produit est au moins 60%, par exemple 80 à 95%.

Le procédé de l'invention peut être effectué de manière continue ou en "batch".

Des molécules ayant une structure proche de celle du bétuloside/rhododendrine peuvent aussi être utilisées dans le procédé de l'invention, par exemple le "méthyl-bétuloside" de formule [3-4'méthoxyphényl)1-méthyl-propyl-β-D-glucopyranoside] (figure 4A), est présent chez <u>Abies webbiana</u>.

La bioconversion de cette substance par le procédé de l'invention donne lieu à la 4-(4'-méthoxyphényl) butan-2-one (voir figure 4B) qui est présente chez <u>Aquilaria agallocha</u>, et qui peut être utilisée en tant que composé d'arôme, ou éventuellement déméthylée pour donner la cétone framboise de l'invention.

L'invention concerne également les compositions de cétone framboise susceptibles d'être obtenues par le procédé enzymatique de l'invention, ainsi que la cétone framboise purifiée. Les compositions de l'invention sont caractérisés par une teneur en cétone framboise d'au moins 50 %, de préférence au moins 80 %, souvent accompagnés de 4-(4'-hydroxyphényl)butan-2-ol (bétuligénol) à des concentrations allant de 0.05 % à 40.0 % environ, typiquement entre 0.5 % et 20.0 %. D'autres composés peuvent également être présents, éventuellement un composé ayant un spectre de masse très voisin de celui de la cétone framboise avec la même fragmentation, mais avec un poids molaire de 150 et dont la formule brute pourrait être $C_9H_{10}O_2$. Il pourrait s'agir de 4-(4'-hydroxyphényl)propan-2-one. La masse des fragments de ce composé et leur abondance sont les suivants : 150 (50), 121 (4), 108 (10), 107 (100) , 77 (42), 45 (30) (voir spectre de masse, Figure 7). La teneur en ce composé est de 0 % à 10 % environ, de préférence entre 0.5 et 5 %. Les spectres de masse des trois composants sont illustrés dans les figures 5, 6, et 7, respectivement

Selon cette variante, le produit de l'invention est une composition aromatique comportant :

i) 50.0 à 99.95 % cétone de framboise et

ii) 0.05 à 40.0 % 4-(4'-hydroxyphényl)butan-2-ol et

iii) 0.0 à 10.0 % d'autres composés provenant de la réaction enzymatique.

De préférence, il s'agit d'une composition comportant :

i) 80.0 a 99.95 % cétone de framboise et

ii) 0.05 à 20.0 % 4-(4'-hydroxyphényl)butan-2-ol.

Les compositions indiquées ci-dessus sont déterminées par analyse par HPLC dans les conditions suivantes :
Colonne Nucléosil C18, 5 microns
Mélange utilisé pour la phase mobile : Eau 60 %, Acétonitrile 40 %,
Conditions isocratiques avec un débit de 0.8 ml / min,
Lecture par spectrophotométrie à 278 nm.

Le produit de l'invention peut aussi être caractérisé par une analyse isotopique. En effet, le produit de l'invention étant normalement issu de matière végétale fraîche, le rapport isotopique C13 / C12 est différent de celui calculé pour une cétone de framboise obtenue par synthèse chimique à partir de matières fossiles.

Le produit de l'invention est utilisé comme substance d'arôme, par exemple "fruits rouges", dans des produits alimentaires, cosmétiques, pharmaceutiques ou dans des parfums. Il peut être utilisé sous toute forme physique normalement employée pour ce type d'application, par exemple dissous dans l'alcool éthylique (96°). On pourrait aussi le mettre en solution dans l'acétate d'éthyle naturel, si le volume de celui-ci est suffisant. Un exemple de formulation dans laquelle la cétone framboise est utilisée pour une application framboise naturelle, est la suivante :

| | |
|---|---|
| Acide acétique naturel | 0.02 |
| Butyrate d'éthyle naturel | 0.02 |
| Acétate d'éthyle naturel | 0.03 |
| γ-Décalactone naturelle | 0.01 |
| Acide butyrique naturel | 0.01 |
| Maltol naturel | 0.10 |
| Cinnamate de méthyle naturel | 0.03 |
| Géraniol naturel (10%) | 0.01 |
| Hexanal naturel | 0.01 |
| Concrète iris | 0.02 |
| Cétone framboise de l'invention | 0.05 |
| Alcool éthylique 96° | 9.69 |
| | 10.00 |

Le produit de l'invention est également utilisable dans d'autres arômes, par exemple : fraise, grenadine, etc.

Différents aspects de l'invention sont illustrés dans les figures :

Figure 1 : montre une variante préférée du procédé enzymatique de l'invention : l'intermédiaire 4-(4'-hydroxyphényl) butan-2-ol est produit par voie enzymatique à partir du 3-(4'-hydroxyphényl)-1-méthylpropyl-β-D-glucopyranoside, l'intermédiaire étant ensuite transformé en 4-(4'-hydroxyphényl)butan-2-one par l'enzyme ayant une activité alcool déshydrogénase.

Figure 2 : les configurations de rhododendrine et d'épi-rhododendrine, et de leurs dérivés aglucones respectifs, le bétuligénol et le rhododendrol.

Figure 3 : schéma de la production de cétone de framboise à partir de rhododendrine (bétuloside) et d'épi-rhododendrine.

Figure 4 : structures chimiques du méthylbétuloside et de la 4-(4'méthoxyphényl)butan-2-one.

Figure 5 : Spectre de masse de la cétone framboise, les valeurs m/z encadrées étant caractéristiques de la cétone framboise.

Figure 6 : Spectre de masse du 4-(4'-hydroxyphényl)butan-2-ol, les valeurs m/z encadrées étant caractéristiques de ce composé.

Figure 7 : Spectre de masse d'un composé ayant un spectre de masse très voisin de celui de la cétone framboise avec la même fragmentation, mais avec un poids molaire de 150 et dont la formule brute pourrait être $C_9H_{10}O_2$. Les valeurs m/z encadrées sont caractéristiques de ce composé. Ce composé peut être présent dans la composition aromatique de l'invention à des concentrations allant jusqu'à 10 %

## EXEMPLES

### EXEMPLE I

1) Extraction du bétuloside à partir d'écorces de bouleau blanc (Betula alba) :

Les écorces de bouleau blanc sont congelées à l'état frais (à -30°C), le plus rapidement possible, par exemple dans la demi-journée après le prélèvement sur l'arbre vivant.

La méthode d'extraction du bétuloside est celle de Sosa A., 1933, C.R. Acad. Sc. Paris, 196 : 1827-1830, modifiée de la manière suivante :

- décoction (eau) à 100°C (2 à 4 l d'eau/kg d'écorce "fraîche") au lieu d'un épuisement de la matière par l'éthanol (à 95°) bouillant ;

- ratio magnésie/écorce : 80g/kg au lieu de 100g/kg.

La concentration en bétuloside retrouvée dans l'écorce (matière fraîche) par cette technique : 1,2% (p/p) ;
le degré de pureté de l'extrait en bétuloside : 13% (p/p) ;
les impuretés présentes aux côtés du bétuloside sont probablement des tanins (tanins catéchiques essentiellement), des sucres, des graisses, des cires, des résines, des phytostérols, des acides gras et des composés condensés divers.

2) Bioconversion du bétuloside en bétuligénol par une β-glucosidase :

L'extrait de bétuloside (8g/l milieu bioréactionnel) obtenu dans la première étape (pureté 13% p/p) est mis en contact avec une β-glucosidase (Emulsine - SIGMA à 6.9 U/mg protéine) à raison de 19.5 mg/g bétuloside, à 37°C dans une enceinte thermostatée. Le volume de tampon acétate (milieu bioréactionnel) (0.2 M, pH 5.0) est de 100 à 300 ml. La réaction se déroule sous agitation magnétique pour une durée de 6 heures.

3) Extraction du Bétuligénol obtenu :

Le bétuligénol obtenu dans l'étape précédente est extrait par l'éther diéthylique : 2 volumes d'éther pour un volume de mélange bioréactionnel, dans une ampoule à décanter. L'opération est réalisée en 2 fois, et est suivie d'une évaporation à sec.

Après ces étapes, le bétuligénol présente une pureté d'environ 30% (p/p).

4) Bioconversion, par microorganisme, du bétuligénol en cétone framboise (en batch) :

Le bétuligénol est mis en contact avec une source d'alcool déshydrogénase - Candida boidinii (DSM 70033). Les conditions opératoires et les rendements de bioconversion sont détaillés dans le tableau A.

**EXEMPLES II à IV**

Les étapes 1 à 3 de l'exemple 1 ont été répétées pour d'autres essais. En général, la concentration des écorces de bouleau en bétuloside atteint 1,2% environ, le degré de pureté de l'extrait en bétuloside étant de 13% (p/p).

Lorsque le bétuloside a une pureté de 15% (p/p), le bétuligénol obtenu après l'étape de bioconversion par la β-glucoside a une pureté d'environ 30% (p/p).

Pour l'étape de bioconversion, par microorganisme, du bétuligénol en cétone framboise (étape 4), les conditions opératoires et les rendements, pour les exemples II a IV, sont détaillés dans le tableau A.

Tableau A

| | | EXEMPLES | | | |
|---|---|---|---|---|---|
| | | I | II | III | IV |
| MICRO-ORGANISME | ESPECE | Candida boidinii (C.b.) | Acetobacter aceti (A. a.) | C. b. | A. a. |
| | N° SOUCHE | DSM 70033 | DSM 3508 | idem I | idem II |
| | DENSITE CELLULAIRE INITIALE | 1 à $2.10^6$ /ml | idem I | idem I | idem I |
| MILIEU | TYPE* | YM | M2 | YM | M2 |
| | pH INITIAL, REGULATION | 5.5 absence | 6.8 absence | 5.5 absence | 6.8 absence |
| | VOLUME | 50 ml | 50 ml | 50 ml | 50 ml |
| PRECURSEUR (BETULIGENOL) | PURETE % (P/P) | 80 | 80 | 20 | 20 |
| | CONC. mg/l CULTURE | 160 | 160 | 200 | 200 |
| | R** | 23 | 25 | 15 | 16 |
| ENCEINTE DE BIOCONVERSION (NATURE, VOLUME) | | ERLEN 150 ml | idem I | idem I | idem I |
| AGITATION AERATION | TYPE, VITESSE | TABLE AGITANTE 110 tr/min | idem I | idem I | idem I |
| | DEBIT D'AIR (l/min) | 0 | 0 | 0 | 0 |
| | CONSIGNE $PO_2$ % | - | - | - | - |
| TEMPERATURE (°C) | | 27 | 27 | 27 | 27 |
| DUREE (h) | | 22 | 34 | 29 | 76 |
| RENDEMENT MOLAIRE MAXIMAL (%) | | 44.5 | 38 | 18 | 15.5 |

**Légende :**

*** Milieux :**

**YM = Extrait de levure : 3g/l**
**Extrait de malt : 3g/l**
**Peptone : 5g/l**
**Glucose : 10g/l**

**M2 = Extrait de levure : 5g/l**
**Peptone : 3g/l**
**Mannitol : 25g/l**

**MC = Extrait de levure : 5g/l**
**Biopolytone : 3g/l**
**Glucose : 27g/l**

**R** = moment d'ajout du précurseur (n$^{ième}$ heure de la culture), début de phase de croissance exponentielle.**

## EXEMPLE V

1) Extraction industrielle du bétuloside à partir d'écorces de bouleau blanc (B. alba) :

a) - Extractions par percolations et macérations à 80°C dans un extracteur de KUMAGAWA.

i) Mouillage et gonflement de l'écorce - percolation 1 ;
170 kg d'écorce de bouleau fraîche, en morceaux, décongelée, sont placés dans l'extracteur puis immergés dans 410 litres d'eau de ville à 60°C. Lorsque la température du solvant a atteint la température de 80°C, on laisse circuler le solvant à travers la plante pendant 2 heures. On laisse ensuite macérer l'écorce dans le solvant, pendant 14 heures sans chauffer. On laisse égoutter le panier pendant 1 heure, on recueille 260 kg d'extrait aqueux à 2,52% de matière sèche.

ii) Percolation - macération 2 ;
On introduit 200 litres d'eau de ville à 60°C dans la cuve de l'extracteur. On procède comme pour i). On recueille 190 kg d'extrait à 1,46% de MS.

iii) Percolation - macération 3 ;
On introduit dans la cuve de l'extracteur 250 litres d'eau de ville à 60°C. On recueille 100 kg d'extrait à 0,81% de MS.

iv) Percolation - macération 4 ;
On introduit dans la cuve de l'extracteur 270 litres d'eau de ville à 60°C. On procède comme pour i). On recueille 250 kg d'extrait à 0,45% de MS.

b) - Traitement des extraits aqueux.

i) Concentration.
Toutes les solutions extractives sont groupées puis concentrées à l'évaporateur industriel en continu. On obtient en final 37,350 kg d'extrait à 27,35% de MS.

ii) Traitement à l'oxyde de magnésium.

On incorpore en agitant 13,6 kg d'oxyde de magnésium (8% du poids d'écorce fraîche), dans les 37,350 kg d'extrait concentré. On laisse sous agitation pendant 2 heures. On obtient 59,950 kg de mélange à 53.27 % de MS. Ce mélange est ensuite séché partiellement à l'évaporateur rotatif sous vide. On termine le séchage à l'étuve a soufflerie à 60°C. L'extrait sec est ensuite broyé finement à l'aide d'un broyeur à couteau.

c) - Extraction secondaire à l'alcool éthylique.

Les 21.7 kg d'extrait sec en poudre sont extraits à reflux pendant 1 heure par 25 litres d'alcool éthylique à 96°. (EtOH 96°/écorce = 59% v/p). L'opération est renouvelée 4 fois. Toutes les solutions extractives éthanoliques sont filtrées sur tissu, groupées, puis séchées partiellement à l'évaporateur rotatif sous vide. On termine le séchage à l'étuve sous vide à 50°C. On obtient en final 872 g d'extrait mou à 67,58% de matière sèche ; pourcentage de bétuloside dans l'extrait mou : 15% (p/p).

d) - Rendement d'extraction.

Matière sèche obtenue : 589 g.
Rendement par rapport à la plante fraîche : 0,35%.
Teneur en eau de l'écorce de bouleau : 24,41%.
Matière sèche dans la plante : 170 -(24,4x170)=128,5kg
Rendement par rapport à la matière sèche : 0,45%
Rendement d'extraction du bétuloside 0,77% ≈ 0,8% (p/p)

2) Bioconversion du bétuloside en bétuligénol par une β-glucosidase :

Les conditions sont celles décrites dans l'exemple I 2) ci-dessus, mais le volume de tampon est de 1.5 à 5 l, et l'enceinte réactionnelle est un fermenteur de 2 a 7 l.

3) Extraction du Bétuligénol obtenu :

Les conditions sont celle décrites dans l'exemple I 3) ci-dessus.

4) Bioconversion, par micro-organisme, du bétuligénol en cétone framboise (en batch) :

Voir tableau B.

**EXEMPLES VI à VIII**

Les étapes 1) à 3) de l'exemple V ont été répétées pour d'autres essais industriels. Les conditions opératoires pour l'étape 4) des exemples VI à VIII sont indiquées dans le tableau B.

Tableau B

| | | EXEMPLES | | | |
|---|---|---|---|---|---|
| | | V | VI | VII | VIII |
| MICRO-ORGANISME | ESPECE | Candida boidinii | Acetobacter aceti (A. a.) | A. a. | A. a. |
| | N° SOUCHE | idem I | idem II | idem II | idem II |
| | DENSITE CELLULAIRE INITIALE | idem I | idem I | idem I | idem I |
| MILIEU | TYPE* | YM | MC | MC | MC |
| | pH INITIAL, REGULATION | 5.5<br>absence | 6.4<br>absence | 6.4<br>absence | 6.4<br>5.5 pendant réaction |
| | VOLUME | 5 l | 5 l | 5 l | 5 l |
| PRECURSEUR (BETULIGENOL) | PURETE % (P/P) | 35 | 35 | 35 | 35 |
| | CONC. mg/l CULTURE | 200 | 200 | 200 | 200 |
| | R** | 10 | 17 | 17 | 17 |
| ENCEINTE DE BIOCONVERSION (NATURE, VOLUME) | | FERMENTEUR<br>7 l | idem V | idem V | idem V |
| AGITATION AERATION | TYPE, VITESSE | TURBINE RUSHTON<br>400 tr/min | idem V | TURBINE RUSHTON<br>700 tr/min | idem V |
| | DEBIT D'AIR (l/min) | 0 à 30 | 0 à 30 | 0 à 30 | 0 à 30 |
| | CONSIGNE $PO_2$ % | 65 | 65 | 80 | 65 |
| TEMPERATURE (°C) | | 30 | 27 | 27 | 27 |
| DUREE (h) | | 32 | 46 | 165 | 93 |
| RENDEMENT MOLAIRE MAXIMAL (%) | | 33 | 35.5 | 23.5 | 86 |

**Légende :**

*** Milieux :**

**YM = Extrait de levure : 3g/l**
**Extrait de malt : 3g/l**
**Peptone : 5g/l**
**Glucose : 10g/l**

**M2 = Extrait de levure : 5g/l**
**Peptone : 3g/l**
**Mannitol : 25g/l**

**MC = Extrait de levure : 5g/l**
**Biopolytone : 3g/l**
**Glucose : 27g/l**

**R** = moment d'ajout du précurseur ($n^{ième}$ heure de la culture), début de phase de croissance exponentielle.**

**EXEMPLE IX**

Les exemples V à VIII ont été répétés avec l'incorporation d'une étape de filtration sur membrane (nanofiltration) de l'extrait d'écorce de bouleau :

Conditions opératoires :

- Appareil : Microlab 80 S GAMMA FILTRATION ;
- Membranes de nanofiltration seuil de coupure 500 ;
- Pression de service 15 bars ;
- Température de régime permanent : 27°C ;
- Surface de membranes 0,1 $m^2$ ;
- Débit avec eau à 20°C ; 54 $l/h/m^2$ ;
- Extrait brut d'écorce de bouleau : 67.58% de MS ;
- Poids total d'extrait mou à 67.58% de MS : 320 g à 15% de pureté (soit 15% de bétuloside) ;
- On prépare 10 litres d'une solution à 1% de MS de l'extrait (soit 160 g d'extrait à 67.58% dans 10 litres d'eau). Une solution à 5% de MS peut aussi être utilisée.

Nanofiltration :

- Débit de perméat départ : 24 ml/min soit 14.4 $l/h/m^2$ ;
- Matière sèche rétentat départ : 0,92% ;

- Matière sèche perméat départ : 0,41% ;

- Lavage à niveau constant avec 12 litres d'eau désionisés : matière sèche perméat 9 l : 0,135% arrêt de la nanofiltration après 12 l de perméat

- Lyophilisation du perméat : produit sec lyophilisé : 10 g reprise du résidu restant sur les plateaux par de l'éthanol à 96, poids : 193 g, MS 14%.

Bilan de la nanofiltration :

- Matière sèche totale : 100 g à 15% de pureté

- Matière sèche totale du perméat : 37 g

- Pureté du produit en admettant que tout le principe actif soit passé à travers la membrane : 40% de pureté théoriquement.

Résultat : bétuloside à 31% de pureté (degré de pureté x 2).

**<u>Matériel et méthodes : Dosage de bétuloside</u>**

La quantification de bétuloside dans les extraits d'écorce de bouleau est effectuée de manière indirecte par dosage par CLHP du bétaligénol libéré après hydrolyse totale du glucoside (bétuloside).

La quantité de bétuligénol représente environ 50% de la quantité du bétuloside (rapport pondéral).

Hydrolyse du bétuloside :

Une solution-mère de A (impur) à 164 g/l dans l'éthanol à 95° est préparée. L'hydrolyse peut être effectuée par voie acide ou par voie enzymatique :

i) Hydrolyse acide :

L'hydrolyse acide est effectuée par l'addition de 0.2 ml de la solution-mère de A (= 3.28 g/l de substrat) à 10 ml d'une solution aqueuse à pH = 1 (ajusté avec HCl). Le tube est bouché, et mis dans un bain-marie à 100°C pendant 4 heures, le mélange étant sous agitation magnétique.

ii) Hydrolyse enzymatique :

L'hydrolyse enzymatique est effectuée par l'addition de 0.2 ml de la solution-mère de A à 10 ml de milieu tampon. Le tube est mis dans un-bain-marie à 37°C, sous agitation magnétique.

Au temps to = 0, 2.5 mg enzyme (Emulsine) à 6.9 U/mg, soit 18U, sont ajoutés. La réaction se poursuit pendant 6 heures.

Le tampon est un tampon acétate (0,2 M) à pH = 5.0.

Dosage du bétuligénol libéré après hydrolyse :

1 ml du milieu réactionnel est soumis à une extraction liquide/liquide avec 2 x 1 ml d'éther diéthylique, suivie de l'évaporation à sec de la phase organique recueillie et la reprise de B dans 1 ml d'éthanol (95°).

Analyse de B par C.L.H.P :

L'appareillage utilisé comporte les éléments suivants :

- chromatographe LC 5000 (Varian)

- Détecteur U.V - 100 (Varian)

- Enregistreur (Model 9176) (Varian)

- Colonne "phase inverse" : Nucléosil C18 Interchrom N5C 18-25 (Interchim longueur 250 mm ; diamètre interne : 4,6 mm, diamètre des particules : 5 µm

Conditions chromatographiques :

- échantillon : 10 µl échantillon éthanolique
- phase mobile : eau - acétonitrile (60 : 40)
- débit : 0.8 ml/min
- température : 35°C
- longueur d'onde λ : 278 nm

Temps de rétention du bétuligénol : 5,6 min.

Ce dosage est basé sur une hydrolyse totale, l'absence de dégradation de B et 100% d'extraction.

**Revendications**

1. Procédé de fabrication, par voie enzymatique, de 4-(4'-hydroxyphényl)butan-2-one ("cétone framboise") caractérisé par la mise en contact du 4-(4'-hydroxyphényl)butan-2-ol avec une source d'enzyme ayant une activité alcool déshydrogénase.

2. Procédé selon la revendication 1 caractérisé en ce que la 4-(4'-hydroxyphényl)butan-2-ol est le (-)-4-(4'hydroxyphényl)butan-2-ol ("bétuligénol").

3. Procédé selon la revendication 1 caractérisé en ce que le 4-(4'-hydroxyphényl)butan-2-ol est le (+)-4-(4'-hydroxyphényl)butan-2-ol ("rhododendrol").

4. Procédé selon l'une quelconque des revendications 1 à 3 caractérisé en ce que la source d'enzyme ayant une activité alcool déshydrogénase est un micro-organisme présentant une activité alcool déshydrogénase, ou un extrait d'un tel micro-organisme, ou une alcool déshydrogénase purifiée à partir de l'un ou plusieurs de ces micro-organismes ou encore un tissu ou extrait végétal présentant une activité alcool déshydrogénase, ou encore un extrait animal, tel qu'un extrait de foie.

5. Procédé selon la revendication 4 caractérisé en ce que la source d'enzyme ayant une activité alcool déshydrogénase comprend soit une bactérie appartenant à l'un des genres suivants : Acetobacter, Gluconobacter, Mycoderma, Pseudomonas, Bacterium, Corynebacterium, Rhodopseudomonas, Astasia, Thermoanaerobium, Clostridium, Methanobacterium, Methylobacterium, Methylococcus, Methylosinus, Lactobacillus, Methanocopusculum, Methanomicrobium, Methanogenium, Thermoanaerobacter, Acinetobacter, Comamonas, Aerobacter, Torulopsis, Escherichia, soit un extrait ou une fraction de bactéries appartenant à l'un ou plusieurs de ces genres.

6. Procédé selon la revendication 4 caractérisé en ce que la source d'alcool déshydrogénase comprend soit une levure appartenant à l'un des genres suivants : Lipomyces, Hansenula, Pichia, Candida, Oospora, Saccharomyces, soit un extrait de levure appartenant à l'un ou plusieurs de ces genres.

7. Procédé selon la revendication 4 caractérisé en ce que la source d'enzyme ayant une activité alcool déshydrogénase comprend un champignon du genre Neurospora ou un extrait d'un champignon de ce genre.

8. Procédé selon la revendication 4 caractérisé en ce que la source d'alcool déshydrogénase comprend un tissu ou extrait végétal, provenant par exemple d'Acer nikoense.

9. Procédé selon l'une quelconque des revendications 1 a 8 caractérisé en ce qu'il comprend en outre une étape d'obtention de 4-(4'-hydroxyphényl)butan-2-ol par la mise en contact du 3-(4'-hydroxyphényl)-1-méthyl-propyl-β-D-glucopyranoside ("bétuloside" ou "rhododendrine") avec une préparation enzymatique ayant une activité β-glucosidase.

10. Procédé selon la revendication 9 caractérisé en ce que le 3-(4'-hydroxyphényl)-1-méthyl-propyl-β-D-glucopyranoside est obtenu à partir de plante(s) appartenant à l'une des familles suivantes : Betulaceae, Ericacea, Saxifragaceae ou à partir de Alnus glutinosa, Taxus baccata, Acer nikoense, Cistus palinhae, Abies webbiana ou Cotyledon wallichii.

**11.** Procédé selon la revendication 9 caractérisé en ce que la préparation enzymatique ayant une activité β-glucosidase comprend un micro-organisme ou un tissu végétal ayant une activité β-glucosidase, un extrait ou un homogénat de micro-organisme ou de tissu végétal, ou une préparation enzymatique purifiée à partir d'un micro-organisme ou d'un tissu végétal.

**12.** Procédé selon l'une quelconque des revendications 9 a 11 caractérisé en ce que l'étape d'obtention du 4-(4'-hydroxyphényl)butan-2-ol et l'étape d'obtention de cétone framboise s'effectuent de manière successive.

**13.** Procédé selon la revendication 12 caractérisé en ce que le 4-(4'-hydroxyphényl)butan-2-ol est purifié avant d'être mis en contact avec la source d'alcool déshydrogénase.

**14.** Procédé selon l'une quelconque des revendications 9 à 11 caractérisé en ce que l'étape d'obtention du 4-(4'-hydroxyphényl)butan-2-ol et l'étape d'obtention de cétone framboise s'effectuent de manière simultanée.

**15.** Procédé selon l'une quelconque des revendications 9 à 14 caractérisé en ce que le bétuloside est obtenu dans le milieu réactionnel à partir d'un précurseur du bétuloside.

**16.** Procédé selon l'une quelconque des revendications 9 à 14 caractérisé en ce qu'il comprend les étapes suivantes :

i) extraction du 3-(4'-hydroxyphényl)-1-méthylpropyl-β-D-glucopyranoside à partir d'une source végétale ;

ii) purification de l'extrait brut du glucoside ainsi obtonu ;

iii) mise en contact de l'extrait purifié du 3-(4'-hydroxyphényl)-1-méthyl-propyl-β-D-glucopyranoside, de préférence en milieu aqueux, avec une préparation enzymatique ayant une activité β-glucosidase ;

iv) extraction du 4-(4'-hydroxyphényl)butan-(2-ol ainsi obtenu, de préférence dans une phase organique ;

v) mise en contact du 4-(4'-hydroxyphényl)butan-2-ol avec une source d'alcool déshydrogénase ;

vi) extraction de la cétone framboise ainsi obtenue.

**17.** Composition aromatique susceptible d'être obtenue par le procédé selon l'une quelconque des revendications 1 à 16, comportant :

i) 50.0 à 99.95 % cétone framboise et

ii) 0.05 à 40.0 % 4-(4'-hydroxyphényl)butan-2-ol et

iii) 0.0 à 10.0 % d'autres composés provenant de la réaction enzymatique.

**18.** Composition selon la revendication 17 comportant :

i) 80.0 à 99.95 % cétone framboise et

ii) 0.05 à 20.0 % 4-(4'-hydroxyphényl)butan-2-ol.

**19.** Utilisation de la composition selon la revendication 17 ou 18 comme substance naturelle d'arôme dans des produits alimentaires, cosmétiques, pharmaceutiques ou dans des parfums.

1)

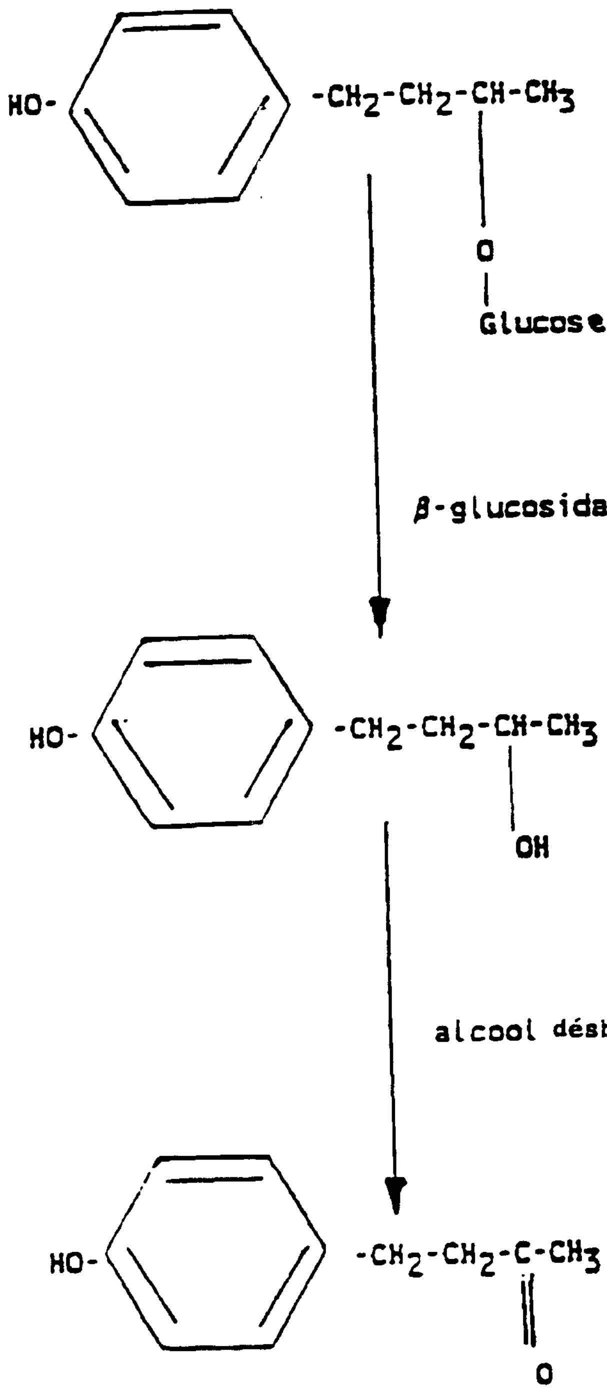


3-(4'-hydroxyphényl)-1-méthyl-propyl-β-D-glucopyranoside

2)

4-(4'-hydroxyphényl) butan-2-ol

3)

4-(4'-hydroxyphényl) butan-2-one

FIGURE 1

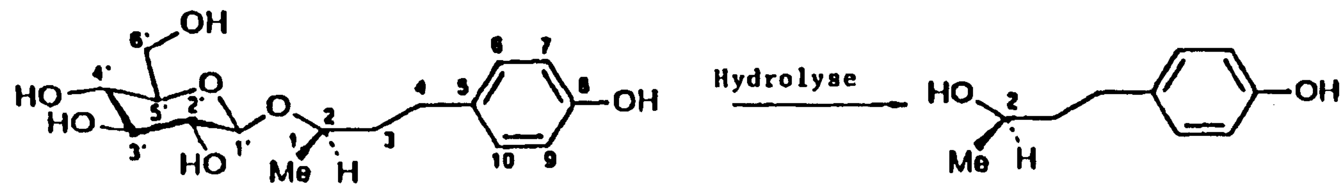


rhododendrine (= bétuloside) (–) -rhododendrol (= bétuligénol)

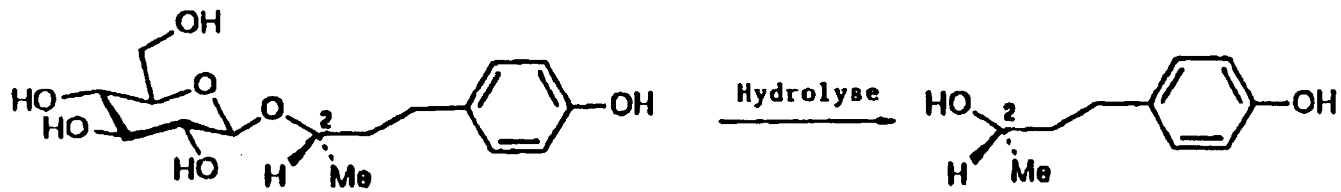


épi-rhododendrine (+) -rhododendrol

FIGURE 2

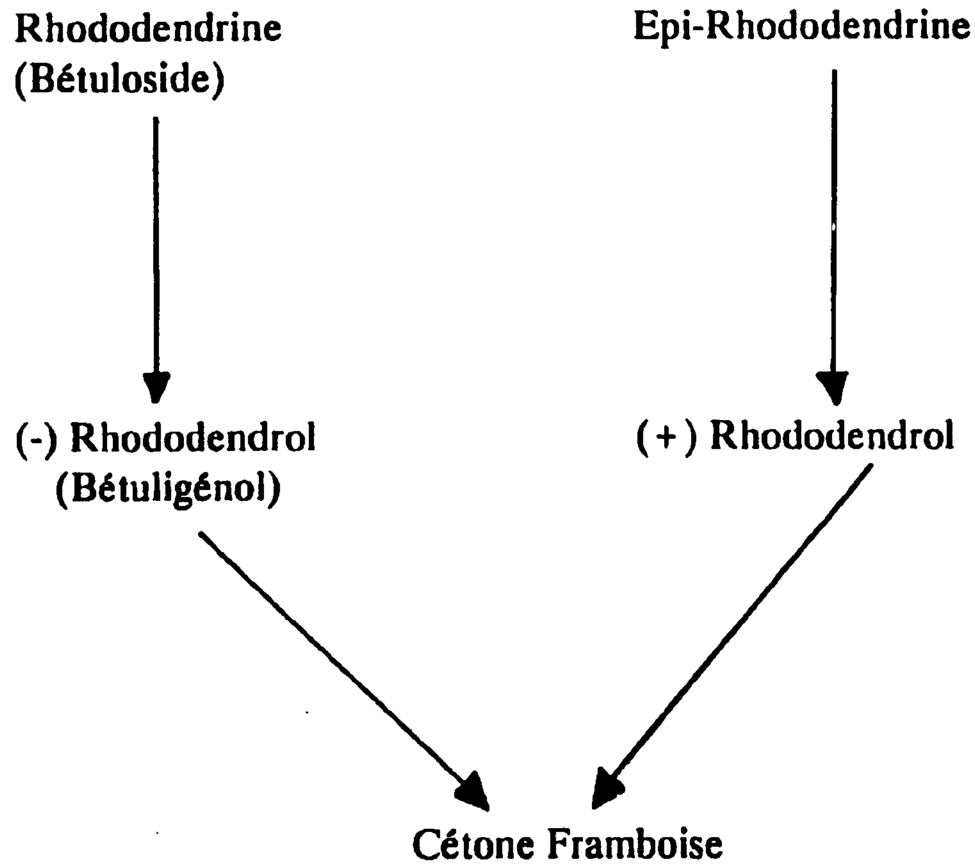
Rhododendrine
(Bétuloside)
Epi-Rhododendrine
(-) Rhododendrol
(Bétuligénol)
(+) Rhododendrol
Cétone Framboise

FIGURE 3

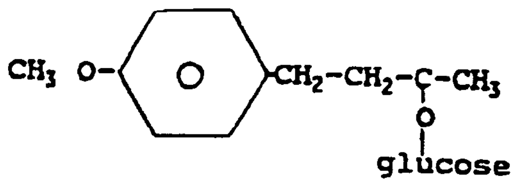

FIGURE 4A

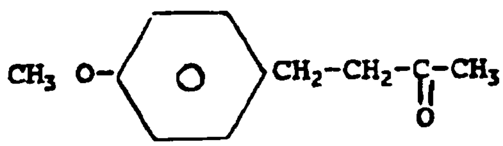

FIGURE 4B

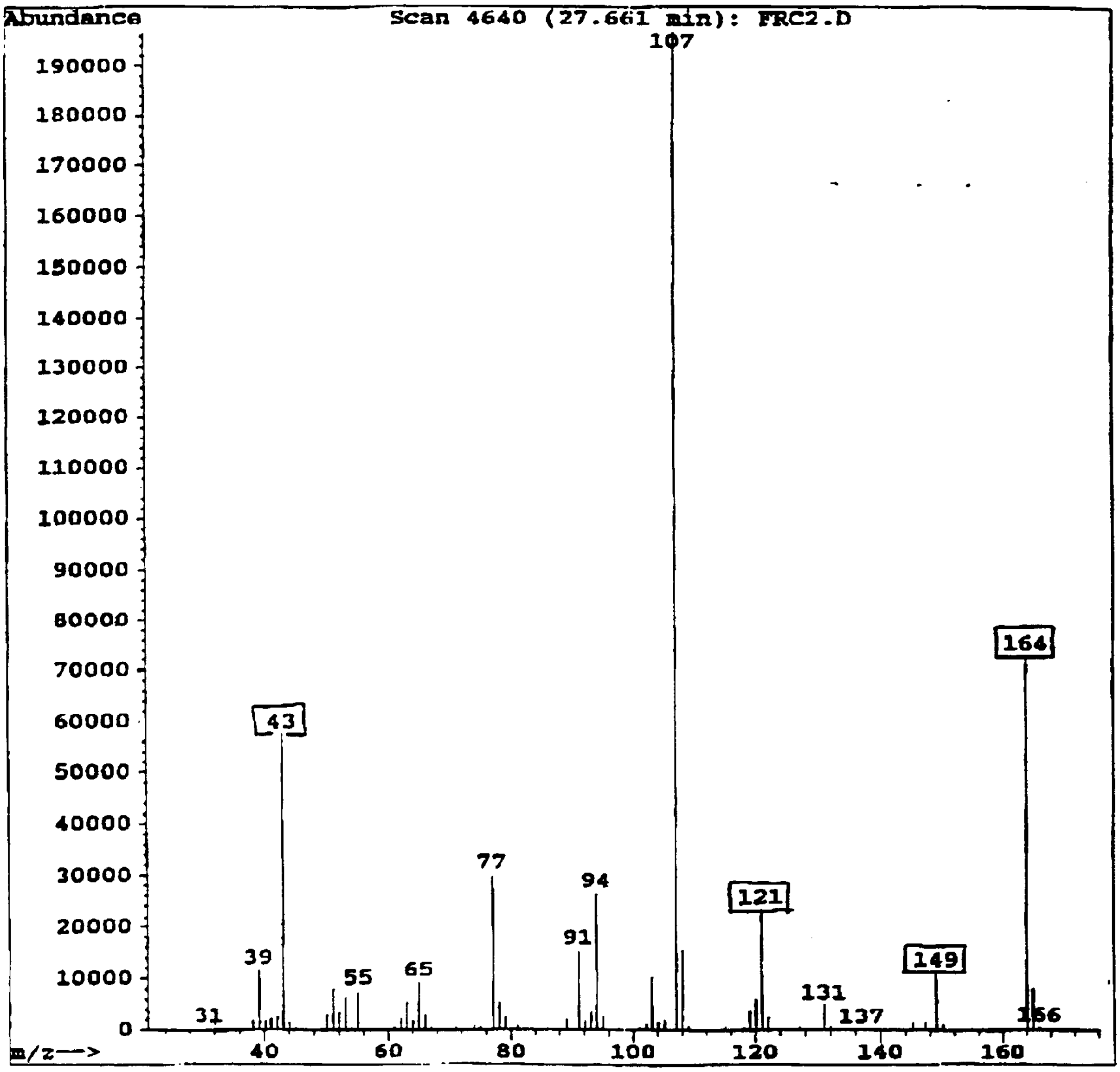
Abundance
Scan 4640 (27.661 min): FRC2.D
190000
180000
170000
160000
150000
140000
130000
120000
110000
100000
90000
80000
70000
60000
50000
40000
30000
20000
10000
0
107
164
43
77
94
121
91
39
149
55
65
131
31
137
166
m/z-->
40
60
80
100
120
140
160

FIGURE 5

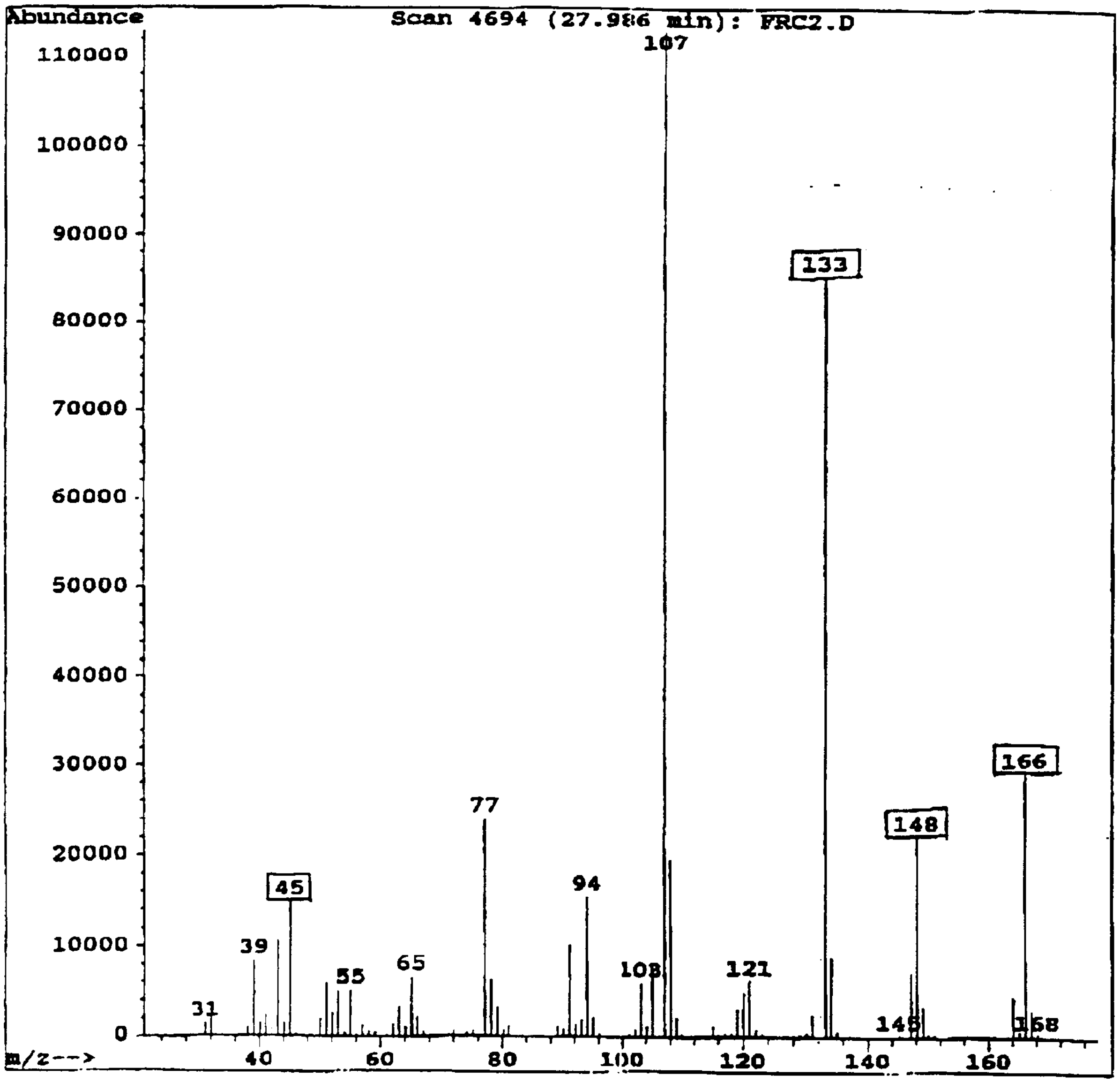
Abundance
Scan 4694 (27.986 min): FRC2.D
110000
100000
90000
80000
70000
60000
50000
40000
30000
20000
10000
0
m/z-->
40
60
80
100
120
140
160
107
133
166
148
77
45
94
39
55
65
103
121
31
145
168

FIGURE 6

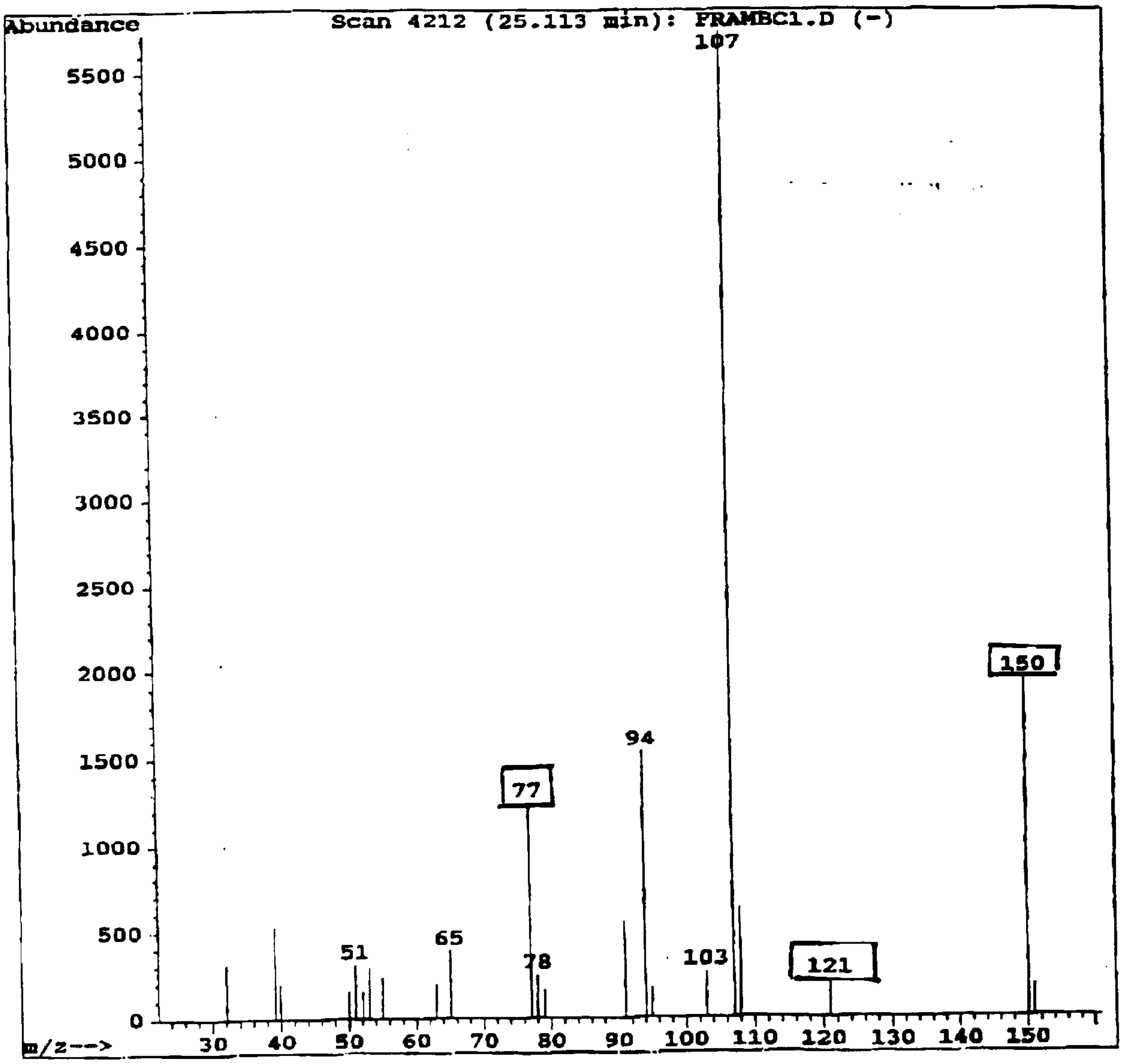
Abundance
Scan 4212 (25.113 min): FRAMBC1.D (-)
107
5500
5000
4500
4000
3500
3000
2500
2000
1500
1000
500
0
150
94
77
65
51
78
103
121
m/z-->
30
40
50
60
70
80
90
100
110
120
130
140
150

FIGURE 7

Office européen des brevets

# RAPPORT DE RECHERCHE EUROPEENNE

Numero de la demande
EP 95 40 2114

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | DATABASE WPI<br>Section Ch, Week 8911<br>Derwent Publications Ltd., London, GB;<br>Class D23, AN 89-081893<br>& JP-A-01 034 941 ( DENKI KAGAKU KOGYO KK) , 6 Février 1989<br>* abrégé *<br>--- | 17-19 | C12P7/26<br>C12P7/22<br>C11B9/00 |
| A | GB-A-2 018 772 (EXXON RESEARCH) 24 Octobre 1979<br>* revendications *<br>--- | 1 | |
| A | US-A-4 241 184 (HOU) 23 Décembre 1980<br>* revendications *<br>--- | 1 | |
| A | US-A-4 250 259 (HOU) 10 Février 1981<br>* revendications *<br>--- | 1 | |
| A | CHEMICAL ABSTRACTS, vol. 119, no. 9, 30 Août 1993<br>Columbus, Ohio, US;<br>abstract no. 93707,<br>FUJITA, TAKASHI ET AL 'Manufacture of (+)-rhododendorol with alcohol dehydrogenase'<br>* abrégé *<br>& JP-A-05 123 175 (SNOW BRAND MILK PROD CO LTD, JAPAN)<br>---<br>-/-- | 1 | DOMAINES TECHNIQUES RECHERCHES (Int.Cl.6)<br>C12P<br>A24B<br>C11B |

Le présent rapport a été établi pour toutes les revendications

| Lieu de la recherche | Date d'achèvement de la recherche | Examinateur |
|---|---|---|
| LA HAYE | 9 Janvier 1996 | Delanghe, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numero de la demande
EP 95 40 2114

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int.Cl.6) |
| A | CHEMICAL ABSTRACTS, vol. 117, no. 11, 14 Septembre 1992 Columbus, Ohio, US; abstract no. 110110, FUJITA, TAKASHI ET AL 'Manufacture of rhododendrol from 4-(p-hydroxyphenyl)-2-butanone with yeast' * abrégé * & JP-A-04 075 587 (YUKI-JIRUSHI NYUGYO K. K., JAPAN) ----- | 1 | |
| | | | DOMAINES TECHNIQUES RECHERCHES (Int.Cl.6) |

Le présent rapport a été établi pour toutes les revendications

| Lieu de la recherche | Date d'achèvement de la recherche | Examinateur |
|---|---|---|
| LA HAYE | 9 Janvier 1996 | Delanghe, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)